**Europäisches Patentamt**

**European Patent Office**

**Office européen des brevets**

(11) Veröffentlichungsnummer: **0 121 583**
**A1**

# (12) EUROPÄISCHE PATENTANMELDUNG

(21) Anmeldenummer: **83103383.2**

(22) Anmeldetag: **07.04.83**

(51) Int. Cl.³: **G 21 F 3/00**

(43) Veröffentlichungstag der Anmeldung: **17.10.84**
**Patentblatt 84/42**

(84) Benannte Vertragsstaaten: **DE**

(71) Anmelder: **Kiy, Otto, Marschallstrasse 67,**
**D-4650 Gelsenkirchen (DE)**
Anmelder: **Plewka, Anna, Nahestrasse 7,**
**D-4300 Essen 18(Kettwig) (DE)**

(72) Erfinder: **Kiy, Otto, Marschallstrasse 67,**
**D-4650 Gelsenkirchen (DE)**
Erfinder: **Plewka, Anna, Nahestrasse 7,**
**D-4300 Essen 18(Kettwig) (DE)**

(54) **Am Kopf zu tragendes Fernsehstrahlen-Schutzgerät für den Zuschauer.**

(57) Bei diesem Schutzgerät handelt es sich um eine mit Sehflächen (4) und Halterung (12) ausgebildete Abdeckung (1) mindestens des den Fernsehstrahlen unterliegenden Kopfbereichs (2) und Halsbereichs (3) gegen gesundheitliche Beeinträchtigungen infolge womöglich austretender schwacher Röntgenstrahlung des Fernsehgeräts.

Technische Probleme wurden im wesentlichen durch folgende Maßnahmen gelöst:

Verwendung von schwache Röntgenstrahlen undurchlässigem Material für die Abdeckung (1), vorwiegend dünne Bleieinlage (21) oder Aluminiumeinlage in einer Kunststoffumhüllung (22), wobei die Sehflächen (4), möglichst aus Kunststoff, eine geeignete Größe, Tönung und Eigenschaft haben;

der die Sehflächen (4) aufweisende untere Teil der Abdeckung (1) hat gegenüber dem die Stirn (5) bedeckenden oberen Teil eine durch Winkel (13) mindestens 2½ cm nach vorn versetzte Ebene auch für den Träger normaler Sehbrille beziehungsweise Spezialbrille und zum freien Atmen und Sprechen.

Die Herstellung des Schutzgeräts in beispielsweise zwei Ausführungen erfolgt mittels Formen beziehungsweise Stanzen von Teilen sowie durch Verschweißen, Zusammenkleben oder auf andere geeignete Art vorwiegend in einem Stück.

0121583

Am Kopf zu tragendes Fernsehstrahlen-Schutzgerät für den
Zuschauer

Die Erfindung betrifft ein am Kopf zu tragendes Fernsehstrahlenschutzgerät für den Zuschauer gegen gesundheitliche Beeinträchtigungen infolge womöglich austretender
schwacher Röntgenstrahlung des Fernsehgeräts.

Anlaß zu der Entwicklung eines solchen Schutzgeräts gaben
die von Fernsehteilnehmern seit einer Reihe von Jahren
empfundenen gesundheitlichen Beeinträchtigungen, insbesondere im Kopf- und Halsbereich, nach regelmäßig langem
Fernsehen. Die Fernsehanstalten haben in der Bundesrepublik Deutschland je Haushalt eine durchschnittliche Fernsehdauer von 2 Stunden und 20 Minuten täglich und in den USA.,
wo in den meisten Haushalten mehrere Apparate vorhanden
sind, von durchschnittlich 6,3 Stunden täglich ermittelt.
Allgemeine deutsche Feststellungen ergaben oft eine Fernsehdauer von über 4 Stunden je Tag und Person. Vorschriften über die Herstellung von Fernsehgeräten lassen einen
Strahlendurchlaß bis 0,5 Milliröntgen je Stunde als Höchstgrenze aufgrund technischer Prüfungen zu.

Ob aus dem Fernsehgerät schwache Röntgenstrahlen überhaupt
austreten können, dürfte sich aus folgendem auszugsweisen
Bericht mit dem Titel "Fernsehkrämpfe" aus der Zeitschrift
"Bleib gesund" des Bundesverbandes der Allgemeinen Ortskrankenkassen, Ausgabe Essen, 1.Quartal 1964, Seite 2,
ergeben (Wirtschaftsdienst Verlag und Druckerei G.m.b.H.,
Frankfurt a.M., Langestr. 13): "Das Flackern einer aus dem
Takt geratenen Bildröhre beim Fernsehen kann schwere epileptische Anfälle verursachen. Besonders auffallend ist
die Tatsache, daß etwa 75 Prozent der Patienten (Kinder)
nur dann Krämpfe bekamen, wenn sie regelmäßig und längere
Zeit dem Fernsehen zusahen." Ferner wurde festgestellt,
daß in der Nähe von Fernsehgeräten beispielsweise auch
Zimmerpflanzen leiden und Zimmervögel mit der Zeit eingehen.

0121583

Über den Stand der Technik ist nichts bekannt.

Das erfindungsgemäße Schutzgerät ist zur Vorsorge besonders für Fernsehstrahlen empfindliche Zuschauer vorgesehen, zu denen auch ältere und kranke Personen sowie Kinder zählen. Außerdem kommen Personen infrage, die sich für ein Fernsehgerät wegen Unverträglichkeit bisher nicht entschließen konnten.

Nachdem der zunächst unbekannte Ursprung der gesundheitlichen Beeinträchtigungen seit Jahren durch eigene und fremde intensive Erforschungen sowie ärztliche Beratung als vom Fernsehen stammend erkannt wurde, dürfte die der Erfindung zugrunde liegende Aufgabe, mittels eines zu entwickelnden einfachen Schutzgerätes diese Beeinträchtigungen von der Person her nicht auswirken zu lassen, zum Nutzen der Fernsehteilnehmer und zum Zwecke der gewerblichen Verwertung als gelöst anzusehen sein.

Im Nachstehenden ist die Erfindung in bevorzugten Ausführungsbeispielen und durch gesonderte Zeichnungen erläutert. Es zeigen:

Fig. 1 schematisch dargestelltes Fernsehstrahlen-Schutzgerät für den oberen Kopfbereich, flach auseinandergelegt

Fig. 2 am Kopf im wesentlichen locker angelegtes Fernsehstrahlen-Schutzgerät nach Fig. 1, von vorn gesehen

Fig. 3 Fernsehstrahlen-Schutzgerät nach Fig. 2, jedoch mit Halsabdeckung, aus seitlicher Sicht (auch Fig. 3 a)

Fig. 4 am Kopf im wesentlichen mit Abstand angelegtes Fernsehstrahlen-Schutzgerät in Schildform für den Kopf- und Halsbereich (auch Fig. 4 a)

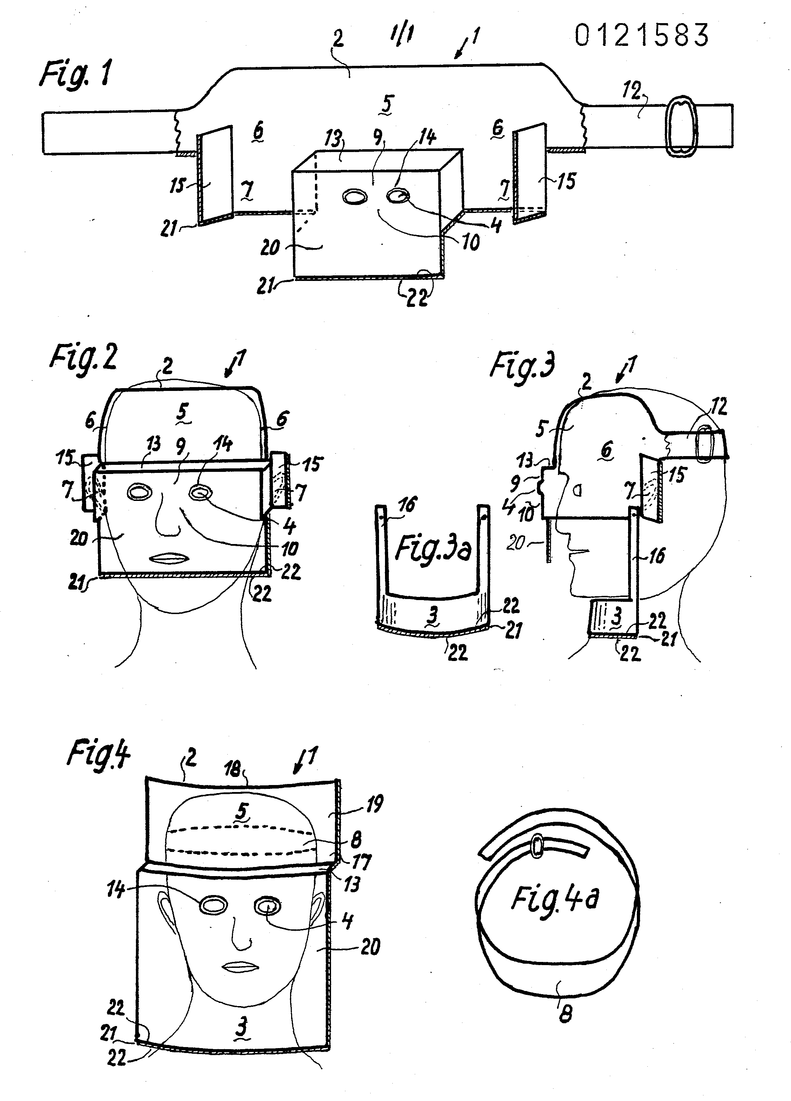

Nach Fig. 1 und 2 hat das Fernsehstrahlen-Schutzgerät hinsichtlich des Kopfbereichs 2 eine im wesentlichen lockere Abdeckung 1 der Stirn 5, der Schläfen 6 sowie der vorderen Gehörzonen 7, fest vereinigt mit einer in breiter Streifenform abstehend ausgebildeten Brille 9, deren Brillenkörper 10 mit der unteren Erweiterung 20 zur Abdeckung des Bereichs um die Augen herum beziehungsweise über das Nasenbein hinaus dient. Dabei ist der Brillenkörper 10 links- und rechtsseitig auch mit den Gehörabdeckungen 7 fest verbunden. Der Brillenkörper sowie die untere, vorzugsweise etwas abgesetzte Erweiterung haben eine zueinander angepaßte und schwach nach hinten gebogene Form. An der oberen Kopfabdekkung 1, nach hinten zu, befindet sich eine Halterung 12 aus einfachem Material und umschließt den Kopf, größenmäßig veränderbar durch Gummizug oder anderweitig. Wegen der oft starken Haarfülle ist bei dieser Ausführung des Schutzgeräts eine lockere Abdekkung vorgesehen.

Der Brillenkörper 10 mit der unteren Erweiterung 20 weist gegenüber der Abdeckung der Stirn 5 eine durch Winkel 13 oder andere Krümmung mindestens 2 1/2 cm nach vorn versetzte Ebene auf, wodurch der Träger normaler Sehbrille beziehungsweise Spezialbrille nicht behindert wird und um ein normales Atmen und Sprechen während des Fernsehens zu erreichen. Das schmale, etwa waagerecht verbleibende Winkelstück kann mit einigen Löchern versehen sein.

Die Sehflächen 4 der Brille 9 sind aus lichtdurchlässigem Kunststoff, haben eine für den Empfang des Bildes sowie die Fernsehstrahlen geeignete Größe, Tönung sowie Eigenschaft und sind je auf verstärktem Rand 14 des Brillenkörpers 10 fest angebracht oder vorzugsweise in dem am Brillenkörper einschiebbar ausgebildeten Metall- oder anderen nicht biegsamen Rahmen angeordnet, damit die Sehflächen nicht verbogen werden können. Jedoch soll bei beispielsweise ovalförmigen Sehflächen die Größe von 32 x 18 mm möglichst nicht unterschritten werden, um stets ein volles Fernsehbild vor Augen zu haben.

An der rückwärtigen Kante beiderseitig der Gehör-

0121583

abdeckung 7, im Winkel zu derselben und flach zum Fernsehgerät ist jeweils ein feststehender scheibenartiger Vorsprung 15 von mindestens 7 cm Höhe und 2 cm Breite angeordnet, aber unter Verwendung einer möglichst harten, nicht umkippbaren Kunststoffumhüllung 22 für die Bleieinlage 21 oder Aluminiumeinlage zur erweiterten, in diesem Falle offenen auch die Ohren einbezogenen Gehörabdeckung 7, wobei eine Beeinträchtigung der Lautstärke auf das Ohr vermieden wird.

Diese Ausführung des Schutzgeräts ist, senkrecht ohne dazwischen befindliche Winkel beziehungsweise Krümmung und ohne Halsabdeckung gemessen, vorn mindestens 18 cm, links - und rechtsseitig je 14 cm hoch.

Die Fig. 3, von der Seite betrachtet, beziehungsweise Fig. 3 a bedeutet eine Erweiterung der in Figur 2 dargestellten Abdeckung 1 auf den Fernsehstrahlen unterliegenden Teil des Halsbereichs 3. Demgemäß ist die Halsabdeckung 3 mindestens 7 cm breit und mit der Abdeckung der Gehörzonen 7 durch schmale Überlappungen 16 fest oder vorwiegend mittels Druckknöpfen beziehungsweise Einrastung abnehmbar verbunden. Die Halsabdeckung kann jedoch, ohne Überlappungen, den Hals beispielsweise durch ein in der Größe veränderbares oder teilbares schmales Band aus einfachem Kunststoff oder Baumwolle nach hinten umschließen.

Aus Fig. 4 und 4 a geht eine weitere Ausführung des Schutzgeräts hervor. Demnach ist am Kopfband 8 vorn eine einteilige Abdeckung 1, mindestens von der Stirn 5 bis zum Hals 3 reichend und über die Kopfbreite 17 des Trägers hinausragend, in Form eines etwa quadratischen, links- und rechtsseitig wenig nach hinten gebogenen Schildes 18 mit Sehflächen 4 fest oder abnehmbar angebracht. Auch hier sind die Metall- oder anderen festen Rahmen 14 vorwiegend für herausnehmbare Sehflächen 4 ausgebildet, zum Beispiel für den Fall, daß eine mitverwendete Spezialbrille bereits getönt ist.

Das Kopfband 8 ist nur an der Stirnfläche 5 etwa 5 cm breit, vorn an der Stirn vorzugsweise aus biegsamem einfachen Kunststoff ohne Blei- oder Aluminiumeinlage hergestellt und nach rückwärts, den Kopf durch eine Halterung 12 schmal umschließend, gleichfalls aus einfachem Material, z.B. Baumwollstoff, gefertigt sowie mit einer Größenverstellung mittels Gummizug oder anderweitig geeignet versehen.

Bei der Abdeckung 1 hat der die Sehflächen 4 aufweisende und bis zum Hals 3 reichende untere Teil gegenüber dem oberen, die Stirn 5 bedeckenden Teil ganzflächig eine durch Winkel 13 oder andere Krümmung mindestens 2 1/2 cm nach vorn versetzte Ebene. Die Winkelränder links-und rechtsseitig können verstärkt werden.

Die Höhe dieses Schutzgeräts beträgt mindestens 26 cm, die Breite mindestens 18 cm.

Für die Abdeckung 1 nach Fig. 1 - 4 wird ganzflächig Fernsehstrahlen beziehungsweise schwache Röntgenstrahlen undurchlässiges hautfreundliches Material verwendet, wozu vorwiegend eine Bleieinlage 21 von mindestens 0,2 mm oder eine Aluminiumeinlage von mindestens 0,3 mm Stärke in einer Kunststoffumhüllung 22 von mindestens 1 mm Stärke vorgesehen ist. Die Herstellung des Schutzgeräts erfolgt jeweils mittels Formen beziehungsweise Stanzen von Teilen sowie durch Verschweißen, Zusammenkleben, Einrasten oder auf andere Art möglichst in einem Stück. Für die Figur 1 - 3 kommt elastischer Kunststoff und für die Figur 4 in Schildform wegen der großen Fläche weniger elastischer Kunststoff infrage. Das Schutzgerät in allen Ausführungen kann an dem oberen, am Kopf anliegenden Teil von innen mit einem auswechselbaren Baumwollstoff ausgelegt sein. Aus optischen Gründen sind für das Schutzgerät mehrfache Farben vorteilhaft. Für Kinder ist ein kleineres Schutzgerät vorgesehen.

0121583

<u>P a t e n t a n s p r ü c h e</u>

1. Am Kopf zu tragendes Fernsehstrahlen-Schutzgerät für den Zuschauer, g e k e n n z e i c h n e t d u r c h eine aus geeignetem Fernsehstrahlen undurchlässigen Material hergestellte, mit Sehflächen (4) und Halterung (12) ausgebildete Abdeckung (1) mindestens des einschlägigen Kopfbereichs (2) und Halsbereichs (3), wobei die Sehflächen, vorwiegend aus lichtdurchlässigem Kunststoff, eine für den Empfang des Bildes sowie die Fernsehstrahlen geeignete Größe, Tönung und Eigenschaft aufweisen und die Halterung den Kopf umschließt, gegen gesundheitliche Beeinträchtigungen infolge womöglich austretender schwacher Röntgenstrahlung des Fernsehgeräts.

2. Fernsehstrahlen-Schutzgerät nach Anspruch 1, dadurch gekennzeichnet, daß die Abdeckung (1) vorwiegend eine – schwache Röntgenstrahlen undurchlässige – Bleieinlage (21) oder Aluminiumeinlage in einer Umhüllung (22) aus biegsamem hautfreundlichen Kunststoff oder insgesamt anderes geeignetes Material aufweist und jeweils mittels Formen beziehungsweise Stanzen von Teilen sowie Verschweißen, Zusammenkleben oder auf andere Art möglichst in einem Stück hergestellt ist.

3. Fernsehstrahlen-Schutzgerät nach Anspruch 1 und 2, dadurch gekennzeichnet, daß die Abdeckung im Kopfbereich mindestens die Stirn (5), die Schläfen (6) und die vorderen Gehörzonen (7) im wesentlichen locker erfaßt, mit einer in breiter Streifenform abstehend ausgebildeten Brille (9) fest vereinigt ist und der Brillenkörper (10) nebst der unteren Erweiterung (20) zur Abdeckung des Bereichs um die Augen herum beziehungsweise über das Nasenbein hinaus dient, wobei die Halterung (12) an der Abdeckung schmal und größenverstellbar ausgebildet ist.

4. Fernsehstrahlen-Schutzgerät nach Anspruch 1 bis 3, dadurch gekennzeichnet, daß der Brillenkörper (10) und die untere Erweiterung (20) gegenüber der Abdeckung der Stirn (5) eine durch Winkel (13) oder andere Krümmung mindestens 2 1/2 cm nach vorn versetzte Ebene auch für den Träger normaler Sehbrille oder Spezialbrille und zum freien Atmen und Sprechen hat.

5. Fernsehstrahlen-Schutzgerät nach Anspruch 1 und 3, dadurch gekennzeichnet, daß die Sehflächen (4) je auf einem verstärkten unbiegsamen Rand (14) des Brillenkörpers (10) fest angebracht oder vorzugsweise in dem am Brillenkörper einschiebbar ausgebildeten Metall- oder anderen unbiegsamen Rahmen angeordnet sind.

6. Fernsehstrahlen-Schutzgerät nach Anspruch 1 bis 3, dadurch gekennzeichnet, daß jeweils an der rückwärtigen Kante der Gehörabdeckung (7), im Winkel zu derselben und flach zum Fernsehgerät ein feststehender scheibenartiger Vorsprung (15) von mindestens 7 cm Höhe und 2 cm Breite angeordnet ist zur erweiterten, offenen Gehörabdeckung.

7. Fernsehstrahlen-Schutzgerät nach Anspruch 1 und 2, dadurch gekennzeichnet, daß der Halsbereich (3) eine mindestens 7 cm breite Abdeckung (1) hat, die nach hinten, links- und rechtsseitig, mit der darüber befindlichen Abdeckung der Gehörzonen (7) durch schmale Überlappungen (16) fest oder vorwiegend abnehmbar verbunden ist, oder die Halsabdeckung den Hals nach hinten schmal und lösbar umschließt.

8. Fernsehstrahlen-Schutzgerät nach Anspruch 1, 2 und 5, d a d u r c h   g e k e n n z e i c h n e t , daß an einem etwa 5 cm breiten aus elastischem Material, vorwiegend einfachem Kunststoff, hergestellten Kopfband (8) mit Halterung (12) eine im wesentlichen mit Abstand ausgebildete Abdeckung (1), von der Stirn (5) bis mindestens zum Hals (3) reichend und über die Kopfbreite (17) des Trägers hinausragend, in Form eines etwa quadratischen, links- und rechtsseitig wenig nach hinten gebogenen Schildes (18) in einem Stück angeordnet ist bei Verwendung einer möglichst nicht biegsamen Kunststoffumhüllung (22) für die Bleieinlage (21) oder Aluminiumeinlage.

9. Fernsehstrahlen-Schutzgerät nach Anspruch 1 und 8, dadurch gekennzeichnet, daß die Abdeckung in Schildform (18) mit ihrem oberen Teil (19) am Kopfband (8), und dasselbe überragend, fest oder durch Einrastung angebracht ist, wogegen der von den Sehflächen (4) bis zum Hals (3) reichende untere Teil (20) ganzflächig eine durch Winkel (13) oder andere Krümmung mindestens 2 1/2 cm nach vorn versetzte Ebene hat.

Fig. 1

Fig. 2

Fig. 3

Fig. 3a

Fig. 4

Fig. 4a

0121583

1/1

Europäisches
Patentamt

**EUROPÄISCHER RECHERCHENBERICHT**

0121583

EP 83 10 3383

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | DE-U-1 817 565 (G. KOPP) <br> * Insgesamt * <br><br> --- | 1 | G 21 F 3/00 |
| Y | GB-A-2 086 210 (S. MOATI) <br> * Seite 1, Zeilen 66-85; Anspruch 1; Abbildung 3 * <br><br> --- | 1,2 | |
| A | US-A-4 024 405 (SZOT) <br> * Spalte 2, Zeilen 14-30; Abbildung 1 * <br><br> --- | 5 | |
| A | US-A-2 391 361 (W.J. STEVENSON) <br> * Seite 1, rechte Spalte, Zeilen 12-21; Abbildung 1 * <br><br> --- | 4 | |
| A | US-A-3 868 727 (PASCHALL) <br><br> ----- | | |

**RECHERCHIERTE SACHGEBIETE (Int. Cl. 3)**

G 21 F 3/00
G 21 F 3/02
G 02 C 7/16
A 61 B 6/10
A 61 F 9/06
A 61 F 9/04

Der vorliegende Recherchenbericht wurde für alle Patentansprüche erstellt.

| Recherchenort <br> DEN HAAG | Abschlußdatum der Recherche <br> 06-07-1983 | Prüfer <br> GIANNI G.L.G. |
|---|---|---|

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82